# EUROPEAN PATENT SPECIFICATION

(11) **EP 4 548 092 B1**
(45) Date of publication and mention of the grant of the patent: **06.05.2026**
(21) Application number: 23738201.5
(22) Date of filing: 21.06.2023
(51) Int. Cl.: G01N 33/04, A01J 5/007, A01J 5/013, A01J 5/04

(54) **ARRANGEMENT FOR ALIGNING A NEEDLE AND A DRY STICK WHEN APPLYING A MILK SAMPLE OF AN ANIMAL**
ANORDNUNG ZUM AUSRICHTEN EINER NADEL UND EINES TROCKENSTIFTES BEIM AUFBRINGEN EINER MILCHPROBE EINES TIERES
AGENCEMENT POUR ALIGNER UNE AIGUILLE ET UN BÂTONNET SEC LORS DE L'APPLICATION D'UN ÉCHANTILLON DE LAIT D'UN ANIMAL

(30) Priority: 29.06.2022 SE 2250808
(43) Date of publication of application: 07.05.2025
(73) Proprietor: DeLaval Holding AB, 147 21 Tumba (SE)
(72) Inventor: CARLSEN, Thomas Nikolai, 147 21 Tumba (SE); GUDMUNDSSON, Kristjan Freyr, 147 21 Tumba (SE); NOROUZI, Ehsan, 147 21 Tumba (SE)
(74) Representative: DeLaval International AB
(86) International application number: PCT/SE2023/050643
(87) International publication number: WO 2024/005694

(56) References cited:
- WO-A1-2017/144913
- WO-A1-2018/236271
- WO-A1-2020/251460

## Description

### TECHNICAL FIELD

This document discloses an arrangement. More particularly, an arrangement is described, arranged to align a needle and a dry stick in order to apply a milk sample of an animal to the dry stick when the dry stick is positioned in a milk sample apply position in which the needle is aligned with the dry stick, wherein the dry stick is configured to indicate at least one biomarker value of the applied milk sample.

### BACKGROUND

On an animal farm, it is important to keep the animals healthy in order to enhance milk/ meat production. On a dairy farm, for example, it is very important to inseminate animals at an optimal moment in order to successfully fertilise the cow. It is important to find the right moment to inseminate each individual animal in the farm, for efficiency reasons. In case the animal is not successfully inseminated, milk production is affected.

Several biomarker measurements may be made on the animal, such as e.g. measuring levels of progesterone, LDH (Lactate Dehydrogenase), BHB (Beta-Hydroxybutyrat) and urea. Thereby important information concerning e.g. heat detection and/ or pregnancy of the individual animal may be made (based on measured progesterone level), as well as mastitis (based on LDH) and ketosis (based on BHB). Also, the energy balance may be estimated (based on urea).

A milk analysis apparatus/ service module may be arranged to cooperate with a milk extracting arrangement, for regularly analysing milk samples of the animals, e.g. at or around the moment of a milking session. The milk analysis apparatus/ service module may extract a milk sample and provide it on a dry stick/ lateral flow stick/ lateral flow test strip, or similar. The milk may be diluted with a diluent, which also may be used to rinse the tubings between test sessions. The optional diluent may be provided in a liquid container. Document WO 2018236271 shows an example of a milk analysis apparatus of this type. Thereby, a farmer/ operator is provided with important information concerning each individual animal. The biomarker such as progesterone may be measured of all individual animals at the farm with a high degree of automation.

An emerging trend is to reduce width of lateral flow sticks in order to save material. Thereby, material is saved and the cost for the dry stick is reduced. 5mm is often regarded as a prior art minimum recommended width of the dry stick. In case the width is more narrowly reduced, for example to 4mm, 3.5mm, 3mm, 2.5mm, 2mm, etc., or when the lateral edge/s is/ are incorrectly cut, for example several, problems may emerge.

A disadvantage is that the narrower width the dry stick has, the more challenging will it be to align the needle with the dry stick and apply the extracted milk sample onto the dry stick, i.e. to direct the needle to the sample pad of the dry stick. In case the milk sample is applied aside of the dry stick, the biomarker measurement will fail.

It would be desired to find a reliable way of applying the milk sample to a dry stick, also when the dry stick is very narrow in relation to the conventional width of about 5mm.

### SUMMARY

It is therefore an object of this invention to solve at least some of the above problems and enable application of a milk sample of an animal to the dry stick also when the dry stick is very narrow, such as for example the dry stick has a width of about 1-3 mm, preferably 2-2.5 mm.

According to a first aspect of the invention, this objective is achieved by an arrangement arranged to align a needle and a dry stick in order to apply a milk sample of an animal to the dry stick when the dry stick is positioned in a milk sample apply position. The dry stick is configured to indicate at least one biomarker value of the applied milk sample.

A biomarker, or biological marker, generally refers to a measurable indicator of some biological state or condition of the animal. The biomarker value measurement may be associated with pregnancy/ reproduction of the animal.

The arrangement comprises a carrier, comprising at least one dry stick. The carrier has a longitudinal extension, extending in a Y- direction. The carrier may for example be embodied as a carrier tape, on which the dry stick, or plurality of dry sticks are arranged. Alternatively, the carrier may be embodied as a blister package.

Also, the arrangement comprises a tube element arranged to receive the milk sample of the animal and the needle. The needle is connected to the tube element, wherein the needle is arranged to receive the milk sample of the animal via the tube element and apply the milk sample to the dry stick.

In addition, the arrangement also comprises a first light source directed to illuminate the dry stick for creating a first shadow of the dry stick on the carrier. The first light source may be embodied as a lamp, or flash.

The arrangement additionally comprises a camera configured to capture an image of at least a part of the dry stick and the first shadow of the part of the dry stick. Furthermore, the arrangement comprises a drive unit comprising an interface configured to cooperate with the carrier in order to move the dry stick on the carrier in the Y- direction to the milk sample apply position.

The arrangement also comprises a controller, communicatively connected to the camera and the drive unit. The controller is configured to trigger the camera to capture the image of the part of the dry stick and the first shadow of the part of the dry stick on the carrier, which first shadow has been created by the first light source. Additionally, the controller is configured to receive the captured image from the camera. The controller is also configured to perform an image analysis of the image, for detecting the part of the dry stick and the first shadow of the part of the dry stick on the carrier. The controller is finally configured to provide an apply milk signal to the drive unit to move the carrier and the dry stick in the Y-direction, into the milk sample apply position based on the image analysis, wherein the needle is enabled to apply the milk sample to the dry stick.

By creating a shadow of the dry stick on the carrier by illuminating the dry stick from the side with the first light source, a sharp contrast is created by the dry stick edge (which typically is white or very light) between the dry stick edge and the carrier. A correct location of the dry stick edge is thereby enabled by image analysis.

In a first implementation of the arrangement according to the first aspect, the controller may be configured to perform the image analysis of the image for detecting a first edge of the dry stick by detecting the first shadow of the first edge of the dry stick, on the carrier.

In a second implementation of the arrangement according to the first aspect, the needle is arranged at a predetermined position in the Y- direction and an X- direction which is perpendicular to the Y-direction. The predetermined position of the needle is known by the controller.

The image analysis of the image, performed by the controller may comprise detecting a section of a first edge of the dry stick by detecting the first shadow of the part of the dry stick on the carrier. The image analysis may also comprise extrapolating an extension of the section of the first edge of the dry stick at a segment of the dry stick where the milk sample is to be applied by the needle when the dry stick is in the milk sample apply position. In addition the image analysis may comprise calculating a distance in the Y-direction between the extrapolated extension of the first edge at the segment of the dry stick and the predetermined position of the needle. The image analysis may also comprise generating the milk apply signal to be provided to the drive unit to move the carrier and the dry stick in the Y-direction into the milk sample apply position, based on the calculated distance thereby aligning the predetermined position of the needle with the section of the dry stick where the milk sample is to be applied by the needle.

The controller is enabled to correct the positioning of the dry stick in the Y-direction, also when the camera is able to capture an image only of a part of the dry stick.

In a third implementation of the arrangement according to the first aspect, or any implementation thereof, the first light source may be directed to illuminate the dry stick at an angle of incidence of substantially 45 ±15 degrees.

The sharp and distinct first shadow of the first dry stick edge created on the carrier in this way by the first light source makes the first edge of the dry stick easily recognisable when running the image analysis.

In a fourth implementation of the arrangement according to the first aspect, or any implementation thereof, the controller may have knowledge of the width of the dry stick. The controller may also be configured to move the carrier and the dry stick in the Y-direction, for aligning the dry stick where the milk sample is to be applied, with the predetermined position of the needle, based on the image processing and with knowledge of the width of the dry stick.

Having determined the correct location of the first dry stick edge and with knowledge of the width of the dry stick, the controller can calculate an adjustment of the dry stick in the Y-direction, for bringing the dry stick into the milk sample apply position where the milk sample is applied via the needle in the middle between the two lateral edges of the dry stick.

In a fifth implementation of the arrangement according to the first aspect, or any implementation thereof, the arrangement may comprise a second light source. The second light source may be directed to illuminate the dry stick for creating a second shadow of a second edge of the dry stick on the carrier. The controller may also be configured to switch off the light of the first light source and ignite the second light source. In addition, the controller may be configured to trigger the camera to capture the image of the second edge of the dry stick and the second shadow of the second edge of the dry stick on the carrier. The controller may be configured to receive the captured image from the camera, and perform the image analysis of the image, for detecting the second edge of the dry stick by detecting the second shadow of the second edge of the dry stick, on the carrier.

The controller may also be configured to move the carrier and the dry stick in the Y-direction, for aligning the dry stick on which the milk sample is to be applied, with the predetermined position of the needle, based on the detected first edge of the dry stick and the second edge of the dry stick, respectively.

By correct positioning of the dry stick and an application of the milk sample symmetrically between the first and second lateral edges of the dry stick, a successful application of the milk sample to the dry stick is assured, reducing risks of applying the milk sample beside the dry stick, also when the dry stick is very narrow, such as for example about 1-3 mm.

In a sixth implementation of the arrangement according to the first aspect, or any implementation thereof, the dry stick has a thickness that extends in a Z-direction, which Z-direction is perpendicular to the Y-direction. The first shadow on the carrier may be created by light from the first light source and the part of the dry stick extending in the Z-direction.

The image analysis of the image may comprise detecting that two or more dry sticks have been stacked onto each other on the carrier in the Z-direction. The image analysis may then comprise the step of estimating extension of the first shadow of the part of the dry stick on the carrier in the Y-direction before the milk sample is applied to the dry stick. In addition the image analysis may comprise comparing the estimated shadow extension with an expected shadow extension value; and, when the estimated shadow extension exceeds the expected shadow extension value, generating a forward signal to be provided to the drive unit to forward the carrier in the Y-direction, to a subsequent dry stick on the carrier.

The dry sticks are rather thin and two dry sticks may sometimes by mistake be stacked one on top of the other on the carrier. The hereby provided quality-check could easily and automatically detect these manufacturing mistakes, and the carrier could be forwarded to the subsequent dry stick before the milk sample is applied.

In a seventh implementation of the arrangement according to the first aspect, or any implementation thereof, the dry stick may comprise a sample pad for receiving the milk sample. Also, the dry stick may comprise a conjugate pad comprising conjugate. The dry stick may in addition comprise an indication zone with a test line and a control line, arranged to indicate the biomarker of the milk sample, based on the conjugate. In addition the dry stick may also comprise an absorbent pad for absorbing the milk sample. The dry stick may also comprise a porous membrane for receiving a capillary flow of the milk sample from the sample pad via the conjugate pad and the indication zone to the absorbent pad, thereby forwarding conjugate dispersed into the milk sample to the indication zone.

The image captured by the camera may depict at least a part of the absorbent pad, and the shadow that is created by the part of the absorbent pad on the carrier when illuminated by the light source. The controller may be configured to detect unsuccessful flowing of the applied milk sample through the porous membrane to the absorbent pad of the dry stick. The detection of unsuccessful milk flow may be made by estimating an extension of the shadow of the absorbent pad in the Y-direction on the carrier, at a determined time period after having applied the milk sample. Also, the detection may comprise comparing the estimated shadow extension of the shadow of the absorbent pad on the carrier with an expected reference shadow value. The detection of unsuccessful milk flow may also comprise the step of discarding the dry stick when the estimated shadow extension of the shadow of the absorbent pad on the carrier is smaller than the expected reference shadow value.

Failed lateral flow tests could hereby be detected and one or several appropriate actions may be triggered, for example discarding the dry stick/ test; generating an alert to the farmer; forwarding the carrier to a next dry stick for repeating the application of the milk sample to the new dry stick, etc.

Other advantages and additional novel features will become apparent from the subsequent detailed description.

### FIGURES

Embodiments of the invention will now be described in further detail with reference to the accompanying figures, in which:
- Figure 1: illustrates an example of an arrangement for measuring a biomarker value of a milk sample of an animal by aligning a needle and a dry stick in order to apply the milk sample of the animal to the dry stick.
- Figure 2A: illustrates an example of a dry stick as regarded in a side view, according to an embodiment.
- Figure 2B: illustrates an example of a dry stick as regarded from above, according to an embodiment.
- Figure 3A: illustrates various components comprised in an arrangement for aligning a needle and a dry stick in order to apply a milk sample of an animal to the dry stick, assisted by a shadow of the dry stick on a carrier.
- Figure 3B: illustrates an image of a dry stick, captured by a camera of the arrangement, according to an embodiment.
- Figure 4A: illustrates a carrier comprising dry sticks, and a shadow on the carrier, created by light from a light source, according to an embodiment.
- Figure 4B: illustrates an image of a part of a dry stick and a shadow thereof, captured by a camera of the arrangement, according to an embodiment.
- Figure 5: illustrates an image of a dry stick arranged on a carrier, and a shadow on the carrier, created by light from a light source, according to an embodiment.
- Figure 6A: illustrates an image of a dry stick arranged on a carrier, and a shadow on the carrier, created by light from a light source, according to an embodiment.
- Figure 6B: illustrates an image of a dry stick arranged on a carrier, and a shadow on the carrier, created by light from a light source, according to an embodiment.

### DETAILED DESCRIPTION

Embodiments of the invention described herein are defined as an arrangement which may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

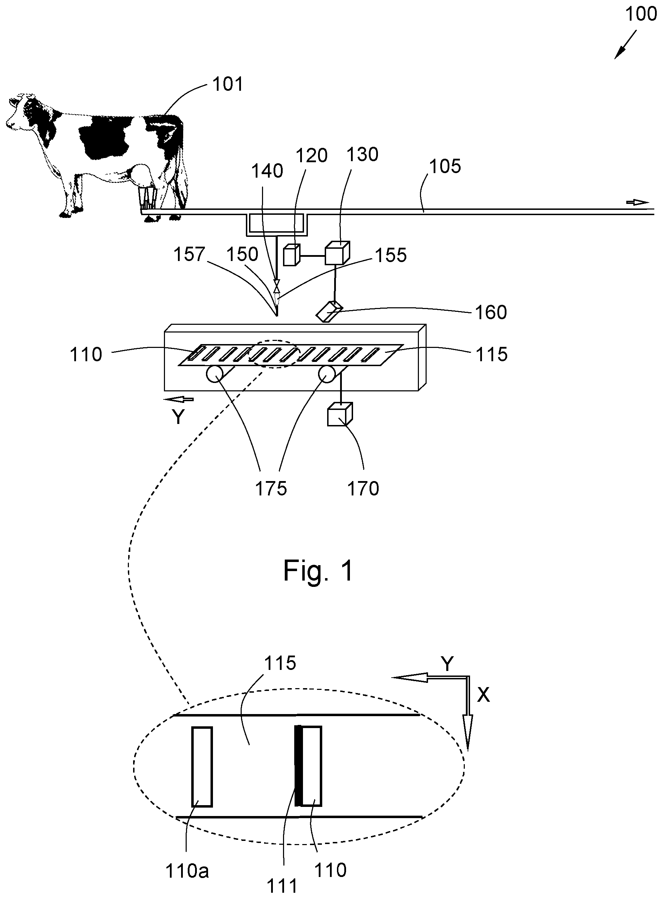

Figure 1 illustrates a scenario with an arrangement 100 arranged to align a needle 150 and a dry stick 110 in order to apply a milk sample of an animal 101 to the dry stick 110 when the dry stick 110 is positioned in a milk sample apply position in which milk sample apply position the needle 150 is aligned with the dry stick 110.

"Animal" may be any arbitrary type of domesticated female milk producing and/ or meat producing mammal such as cow, goat, sheep, horse, camel, dromedary, dairy buffalo, donkey, reindeer, yak, etc.

The lateral flow stick 110 may be individually arranged on a carrier 115 comprising a plurality of lateral flow sticks 110, 110a. Each one of the individually arranged lateral flow sticks 110, 110a on the carrier 115 may be arranged separated from each other.

The dry stick 110 is configured to indicate at least one biomarker value of the applied milk sample.

The carrier 115 has a longitudinal extension, extending in a Y- direction and a transverse extension, extending in an X- direction, wherein the Y- direction and the X- direction are perpendicular to each other.

Each one of the dry sticks 110, 110a has a longitudinal extension and may be arranged on the carrier 115 so that the longitudinal extension of the dry stick 110 extends on the carrier 115 along the X- direction with an inclination within a range of ±45 degrees in relation to the X- direction.

The carrier 115 may be embodied e.g. as a carrier tape, or a blister package. In embodiments wherein the carrier 115 is embodied as a carrier tape, the carrier 115 may be comprised in a cassette, for a swift handling.

Each dry stick 110, 110a may be maintained and sealed individually on the carrier 115 when embodied as a carrier tape, for example sealed by a foil covering the dry stick 110, 110a and the carrier 115. The foil may be made of transparent plastic/ elastomere, for example. In these embodiments, the foil may be peeled off before the milk/ liquid sample is applied to the dry stick 110, 110a. In case the carrier 115 is embodied as a blister package, the seal cover of the blister package may be punctured before application of the milk/ liquid sample.

Milk of the animal 101 may be extracted and evacuated by a milking equipment that are part of a milking parlour via a milk line to a milk tank or similar milk storage.

During milking of the animal 101, or in close relation therewith, the milk sample may be diverted from the milking equipment/ milk line 105 and provided to a tube element 155, having an interface to the milking equipment/ milk line 105. A pump may be configured to act on the tube element 155, to forward the milk sample, from the milking equipment/ milk line 105, via a valve 140 and a mixing chamber, to a needle 150.

In some embodiments, the milk sample may be mixed with a diluent in the mixing chamber, thereby forming a liquid sample comprising a mixture of milk and diluent.

The needle 150 is connected to the tube element 155, possibly via the valve 140 and the mixing chamber. The needle 150 is arranged to receive the milk sample of the animal 101 and apply the milk sample to the dry stick 110.

The needle 150 may be arranged at a predetermined position in the Y- direction and in the X- direction.

The arrangement 100 also comprises a first light source 160, directed to illuminate the dry stick 110 for creating a first shadow 111 of the dry stick 110 on the carrier 115.

The first light source 160 may be directed to illuminate the dry stick 110 at an angle of incidence of substantially 45 ±15 degrees. The first light source 160 may comprise a lamp arranged with a predetermined angle of incidence, with a predetermined illuminance. Illuminance is the total luminous flux incident on a surface, per unit area. The first light source 160 may thereby be applied to ensure proper illumination of the dry stick 110 and create a distinguished first shadow 111 of the dry stick 110 on the carrier 115.

The arrangement 100 in addition comprises a camera 120 configured to capture an image of at least a part of the dry stick 110 and the first shadow 111 of the part of the dry stick 110 on the carrier 115.

The arrangement 100 also comprises a drive unit 170 comprising an interface 175 configured to cooperate with the carrier 115 in order to move the dry stick 110 on the carrier 115 in the Y- direction to the milk sample apply position.

The drive unit 170 may comprise a motor such as a linear motor or engine such as an electric engine in some embodiments.

In case the carrier 115 is embodied as a carrier tape, the carrier tape may be arranged on a first spool and a second spool, possibly on a cassette. The interface 175 may then comprise one or two engaging wheels, arranged to rotate the first and/ or the second spool, thereby causing the carrier 115 to move.

The arrangement 100 also comprises a controller 130, communicatively connected to the camera 120 and the drive unit 170, possibly also to the first light source 160.

The controller 130 is configured to perform a number of actions for aligning the needle 150 and the dry stick 110. The controller 130 is configured to trigger the camera 120 to capture an image of the part of the dry stick 110 and the first shadow 111 of the part of the dry stick 110 on the carrier 115. The first shadow 111 is created by the light of the first light source 160. The controller 130 is also configured to receive the captured image from the camera 120. The controller 130 is also configured to perform an image analysis of the image, for detecting the part of the dry stick 110 and the first shadow 111 of the part of the dry stick 110 on the carrier 115.

The first shadow 111 is created on the carrier 115, thereby creating a sharp and easily recognisable contrast, enabling recognition of a part of the dry stick 110 where the milk sample may be applied via the needle 150 by the image analysis.

Based on the image analysis, the controller 130 is configured to provide an apply milk signal to the drive unit 170 to move the carrier 115 and the dry stick 110 in the Y-direction via the interface 175, into the milk sample apply position, wherein the needle 150 is enabled to apply the milk sample to the dry stick 110.

The needle 150 may be arranged at a predetermined position in the Y-direction and in the X-direction, which predetermined position is known by the controller 130. The controller 130 is thereby enabled to align the needle 150 and the dry stick 110 and apply the milk sample to the dry stick 110 via the needle 150 when the dry stick 110 is positioned in the milk sample apply position.

The dry stick 110 may react on presence and/ or amount of one or several biomarkers, e.g. by changing colours, colour tint or intensity of a colour/ tint. The camera 120 may capture an image of the dry stick 110. The captured image of the dry stick 110 may then be analysed by the controller 130, and based on the intensity of the colour, presence and/ or quantity of the biomarker in the milk sample may be estimated.

The measured biomarker may be e.g. progesterone, glycoprotein, oestrogen and/ or Gon-adatropin-Releasing Hormones, or any other similar biomarker associated with reproduction of the animal 101, in different embodiments.

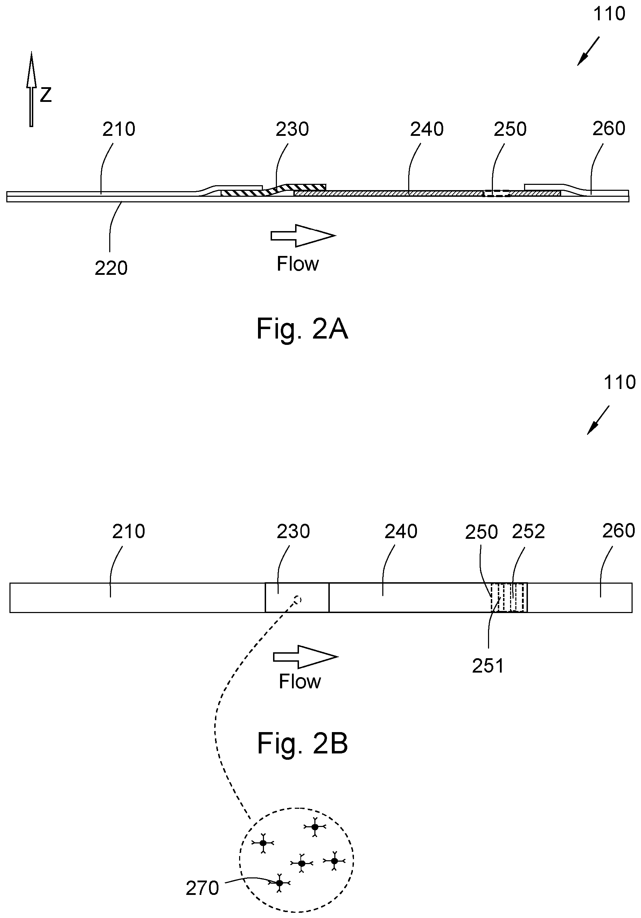

Figure 2A illustrates a dry stick 110 as regarded from a side view while Figure 2B illustrates the dry stick 110 as regarded from above.

The dry stick 110 may comprise a back plate 220, which may be made of cardboard, paper or similar material. The back plate 220 may form a base on to which other components of the dry stick 110 may be mounted.

The dry stick 110 may comprise a sample pad 210. The sample pad 210 is the part of the dry stick 110 onto which the milk sample/ liquid sample is applied by the needle 150 when the dry stick 110 is situated in the milk sample apply position. The sample pad 210 may comprise a porous structure for enabling capillary flow such as cellulose fibres and/ or woven meshes.

The dry stick 110 may also comprise a conjugate pad 230 comprising conjugate 270. The conjugate 270 may comprise antibody treated gold particles which are dispersed into the milk when the milk sample/ liquid sample flows from the sample pad 310 through the conjugate pad 230.

The conjugate 270 will react with the biomarker to be measured, for example progesterone in the milk of the applied liquid sample.

The conjugate 270 may thus be embedded in the conjugate pad 230 which may comprise a glass-fibre section of the dry stick 110. Alternatively, the conjugate pad 230 may comprise cellulose and/ or surface modified polyester.

Also, the dry stick 110 may comprise an indication zone 250, arranged to indicate the biomarker of the milk sample/ liquid sample, based on the conjugate 270. The dry stick 110 furthermore may comprise a porous membrane 240 for receiving a capillary flow of the milk sample/ liquid sample from the conjugate pad 230, thereby forwarding conjugate 270 dispersed into the milk sample/ liquid sample to the indication zone 250.

The porous membrane 240 may comprise for example a nitrocellulose membrane, cellulose, glass fibre, polyester, rayon, a polymer, glass fibre, woven fibres, non-woven fibres, a chromatographic gel membrane, diatomaceous earth, silica gel, silicium oxide, kieselguhr, or other filtration membranes in different embodiments. The porous membrane 240 may be designed to enhance capillary pumping speed of liquid through the dry stick 110. The indication zone 250 of the porous membrane 240 may comprise a test line 251 and a control line 252.

The test line 251 may be treated with a biomarker reference such as e.g. a progesterone reference which binds the antibody treated gold particles of the milk sample/ liquid sample and thereby brings the test line 251 to change colour tint when exposed for milk comprising a progesterone level lower than a threshold limit. Thus, the test line 251 may change colour tint into red/ reddish when the milk has no or low progesterone level. If/ when the animal 101 is in heat, the progesterone level is near zero in the milk sample. This colour change may then be detected by the camera 120 and may be reported to the farmer and/ or stored in a database associated with an identity of the animal 101, and/ or a time stamp. This may also trigger scheduling of insemination of the animal 101 (in case the biomarker is progesterone), or the scheduling of a veterinary check-up, for example (in case of another biomarker).

The control line 252 in the indication zone 250 of the porous membrane 240 may be treated with an antibody reference which binds antibody reference treated gold particles of the milk sample/ liquid sample regardless of the progesterone level in the milk, and may thereby bring the control line 252 to change colour tint when exposed for milk comprising antibody reference treated gold particles, assuring a successful capillar flow of the milk/ liquid through the porous membrane 240.

The dry stick 110 may comprise an absorbent pad 260. The absorbent pad 260 may comprise an absorbent configured to absorb superfluous milk/ liquid from the porous membrane 240.

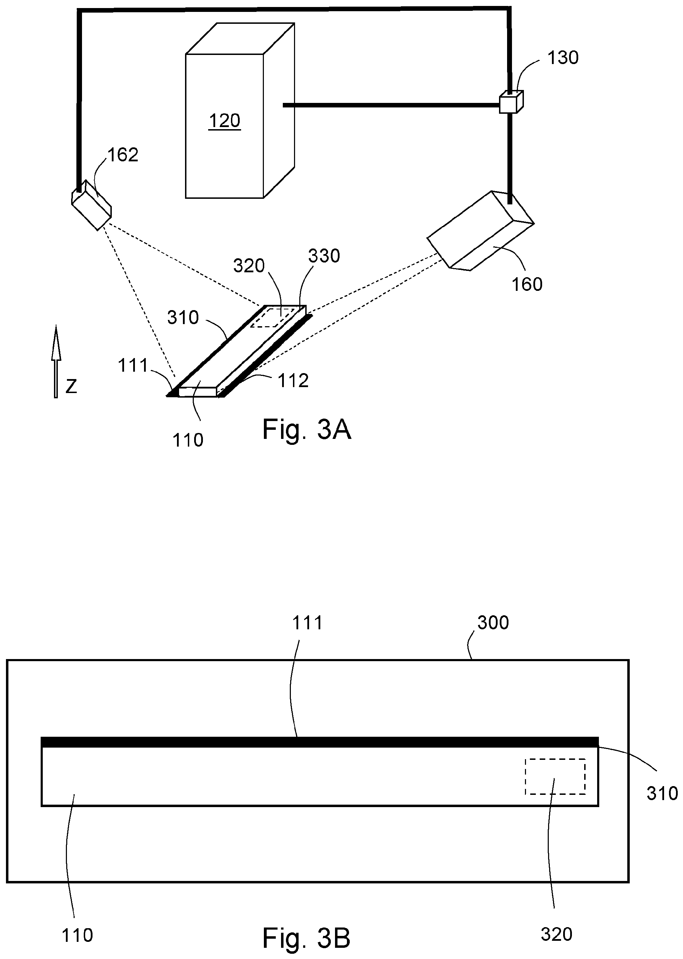

Figure 3A schematically illustrates some elements comprised in the arrangement 100, such as the camera 120, the controller 130, and the first light source 160 illuminating the dry stick 110.

The dry stick 110 has a thickness that extends in a Z-direction. The Z-direction is perpendicular to the Y-direction and/ or the X- direction. The first shadow 111 of the part of the dry stick 110 on the carrier 115 is created by light from the first light source 160 and a first edge 310 of the part of the dry stick 110 extending in the Z-direction.

The controller 130 may be configured to perform the image analysis of the image by a number of actions.

The controller 130 may be configured to detect a section of the first edge 310 of the dry stick 110 by detecting the first shadow 111 of the first edge 310 of the dry stick 110 extending in the Z-direction on the carrier 115.

In some embodiments, the arrangement 100 may comprise a second light source 162 directed to illuminate the dry stick 110 so that a second edge 330 of the dry stick 110 creates a second shadow 112 of the dry stick 110 on the carrier 115, while the first light source 160 is switched off. The second light source 162 may then illuminate the dry stick 110 from an opposite side of the dry stick 110 in relation to the first light source 160.

The controller 130 is thereby enabled to detect both the lateral edges 310, 330 of the dry stick 110 and is thereby enabled to apply the milk sample via the needle 150 to the segment 320 of the dry stick 110 where the milk sample is to be applied in between the first and the second lateral edges 310, 330 of the dry stick 110, also when the controller 130 does not know the width of the dry stick 110, or when different dry sticks 110 have different widths.

The second light source 162 may be directed to illuminate the dry stick 110 at an angle of incidence of substantially 45 ±15 degrees, similar to the first light source 160, but from the opposite lateral side of the dry stick 110.

Figure 3B illustrates an image 300 of the dry stick 110, and the first shadow 111 of the first edge 310 captured by the camera 120.

The sharp and distinguished first shadow 111 created by the first edge 310 of the dry stick 110 on the carrier 115, makes it easy for the controller 130 to determine position of the edge 310, which in turn enable precision when aligning the needle 150 with the segment 320 of the dry stick 110 where the milk sample is to be applied by the needle 150 when the dry stick 110 is in the milk sample apply position, also when the dry stick 110 is very narrow, such as in an interval of about 1-3 mm, preferable 2-2.5 mm, or there about.

The controller 130 may have knowledge of the width of the dry stick 110, for example 2 mm. The width of the dry stick 110 may be manually entered by the operator or estimated by image analysis of the image 300 captured by the camera 120. Having determined the correct location of the dry stick edge 310 and with knowledge of the width of the dry stick 110, the controller 130 can move the carrier 115 and the dry stick 110 in the Y-direction, for aligning the segment 320 of the dry stick 110 where the milk sample is to be applied, with the predetermined position of the needle 150, based on the image processing and with knowledge of the width of the dry stick 110. Thereafter, the controller 130 may apply the milk sample via the needle 150 at a distance corresponding to the width of the dry stick 110 divided by 2, from the first dry stick edge 310.

However, the controller 130 may also apply the herein described methodology on a dry stick 110, having a width of at least 5 mm. Hereby, enhanced successfulness concerning application of the milk sample to the dry stick 110 is achieved.

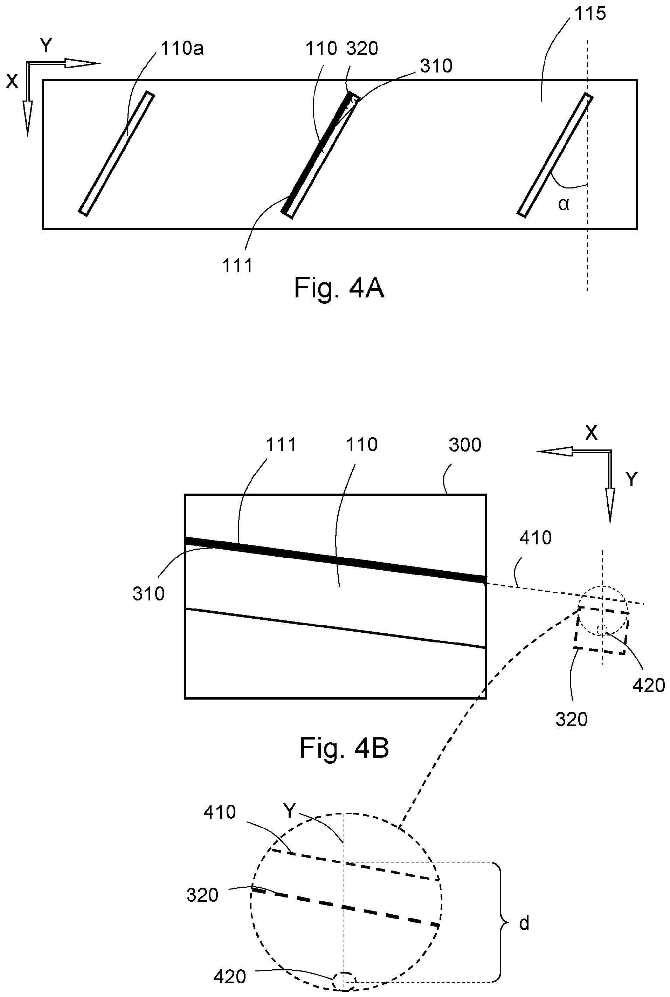

Figure 4A illustrates an example of the carrier 115 as regarded from above. The dry sticks 110, 110a on the carrier 115 are arranged with an inclination α within a range of ±45 degrees in relation to the X- direction (non-limiting example).

Figure 4B illustrates an example of an image 300 captured by the camera 120.

In this example, only a part of the dry stick 110 is captured in the image 300.

The controller 130 may be configured to detect a section of a first edge 310 of the dry stick 110 in the image 300, by detecting the first shadow 111 on the carrier 115, of the part of the dry stick 110 extending in the Z-direction.

The controller 130 may be configured to extrapolate an extension 410 of the first edge 310 of the section of the dry stick 110 at a segment 320 of the dry stick 110 where the milk sample is to be applied by the needle 150 when the dry stick 110 is in the milk sample apply position.

Then, the controller 130 may calculate a distance d in the Y-direction between the extrapolated extension 410 of the first edge 310 and a predetermined position 420 in the Y- direction and the X- direction, of the needle 150.

The controller 130 may also be configured to generate the milk apply signal to be provided to the drive unit 170 to move the carrier 115 and the dry stick 110 in the Y-direction into the milk sample apply position. The milk apply signal and the size of the movement in the Y-direction may be based on the calculated distance d. Thereby, the predetermined position 420 of the needle 150 in the Y- direction and the X- direction is aligned with the segment 320 of the dry stick 110 where the milk sample is to be applied by the needle 150.

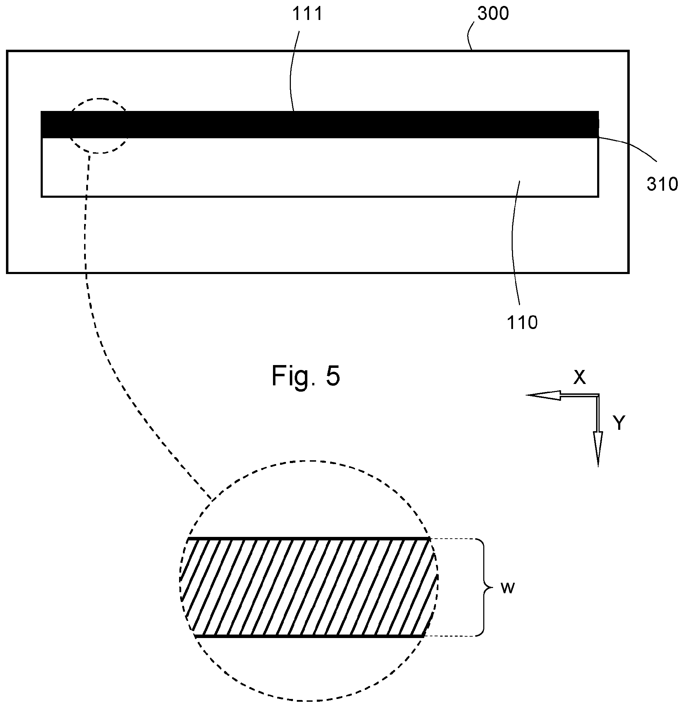

Figure 5 illustrates an example of an image 300 captured by the camera 120, depicting the dry stick 110.

A first edge 310 of the dry stick 110 creates a first shadow 111 on the carrier 115. The first shadow 111 enable a very precise positioning of the first edge 310 by the controller 130. The first shadow 111 created by the dry stick 110 has an extension w in the Y-direction.

The image analysis may in some embodiments comprise detecting that two or more dry sticks 110 by mistake have been stacked onto each other on the carrier 115, in the Z-direction. The controller 130 may be configured to estimate extension w of the shadow 111 on the carrier 115, of the part of the dry stick 110 in the Y-direction before the milk sample is applied to the dry stick 110.

The controller 130 may also be configured to compare the estimated shadow extension w on the carrier 115 with an expected shadow extension value. The expected shadow extension value typically corresponds with the shadow extension w of the shadow 111 of a single dry stick 110 on the carrier 115. When the estimated shadow extension w exceeds the expected shadow extension value, it may at least implicitly be concluded that two or more dry sticks 110 have been stacked onto each other; or possibly that some other non-desired object has accidently been placed on/ under the dry stick 110. In any way, it is inappropriate to use the dry stick 110 for making tests such as lateral flow tests.

The controller 130 may also be configured to generate a forward signal to be provided to the drive unit 170 to forward the carrier 115 in the Y-direction, to a subsequent dry stick 110a on the carrier 115.

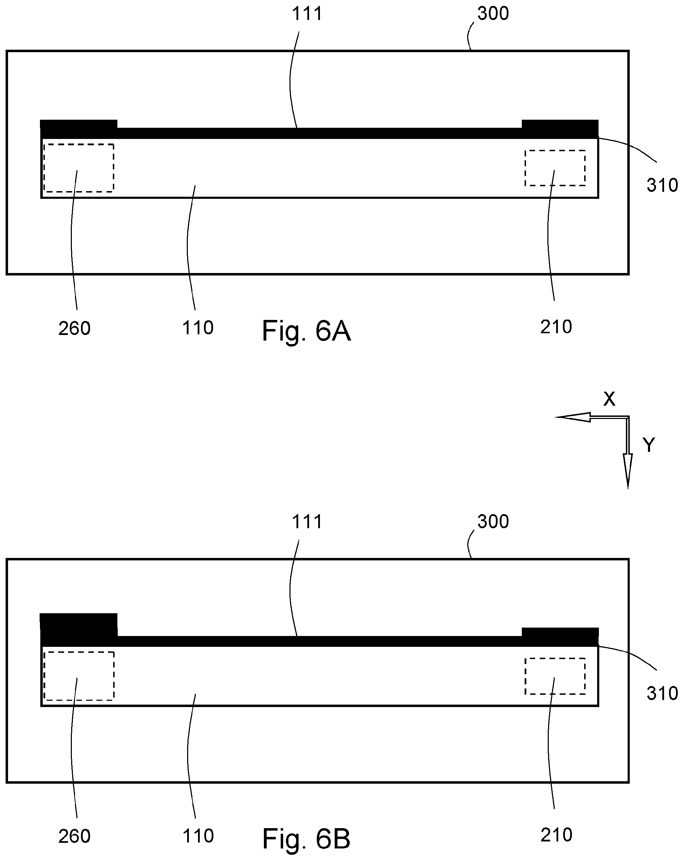

Figure 6A illustrates an image 300 depicting a dry stick 110 comprising a sample pad 210 for receiving the milk sample via the needle 150, before application of a milk sample. The illustrated dry stick 110 also comprises an absorbent pad 260, for absorbing the milk sample.

The dry stick 110 may for example comprise a lateral flow stick as illustrated in Figures 2A-2B and discussed in the corresponding section of the specification.

Figure 6B illustrates an image 300 depicting the dry stick 110 and a shadow 111 of the dry stick 110 on the carrier 115, after application of the milk sample to the sample pad 210, and after the milk sample having propagated by a capillary flow from the sample pad 210 via the conjugate pad 230 and the indication zone 250 to the absorbent pad 260 of the dry stick 110.

The controller 130 may in some embodiments be configured to detect unsuccessful flowing of the applied milk sample through the porous membrane 240 to the absorbent pad 260 of the dry stick 110.

The detection of unsuccessful flowing may comprise estimating an extension w of the shadow 111 on the carrier 115, of the absorbent pad 260 in the Y-direction, at a determined time period after having applied the milk sample.

The determined time period may correspond with an estimated propagation of milk flowing through the dry stick 110 to the absorbent pad 260, after having applied the milk sample to the sample pad 210.

Also, the controller 130 may be configured to compare the estimated shadow extension w of the shadow 111 on the carrier 115, of the absorbent pad 260, with an expected reference shadow value.

The reference shadow value may approximately correspond with a shadow extension of the shadow 111 of the absorbent pad 260 when milk/ liquid has been absorbed by the absorbent pad 260, after the determined time period.

The controller 130 may be configured to discard the dry stick 110 when the estimated shadow extension w of the shadow 111 on the carrier 115, of the absorbent pad 260 is smaller than the expected reference shadow value.

The controller 130 may be embodied as a processing circuitry configured for performing various calculations for conducting various computer programs, for example to communicate with the camera 120 and/ or the drive unit 170, respectively, and/ or to perform the image analysis.

The processing circuitry may comprise one or more instances of a processing circuit, i.e. a Central Processing Unit (CPU), a processing unit, a processing circuit, a processor, an Application Specific Integrated Circuit (ASIC), a microprocessor, or other processing logic that may interpret and execute instructions. The herein utilised expression "processing circuitry" may thus represent a plurality of interconnected and cooperating processing circuits, such as, e.g., any, some or all of the ones enumerated above.

Furthermore, the controller 130 may comprise a memory in some embodiments. The optional memory may comprise a physical device utilised to store data or programs, i.e., sequences of instructions, on a temporary or permanent basis. According to some embodiments, the memory may comprise integrated circuits comprising silicon-based transistors. The memory may comprise e.g. a memory card, a flash memory, a USB memory, a hard disc, or another similar volatile or non-volatile storage unit for storing data such as e.g. ROM (Read-Only Memory), PROM (Programmable Read-Only Memory), EPROM (Erasable PROM), EEPROM (Electrically Erasable PROM), etc. in different embodiments.

Further, the controller 130 may comprise a signal transmitter. The signal transmitter may be configured for transmitting signals via a wired or wireless communication interface to the camera 120, the drive unit 170 and/ or the needle 150 possibly via a transceiver; and/ or to the database.

Furthermore, a computer program comprising instructions to perform the image analysis of the image 300 captured by the camera 120 may be performed in the controller 130.

The computer program mentioned above may be provided for instance in the form of a computer-readable medium, i.e. a data carrier carrying computer program code for performing at least some of the computer program steps, according to some embodiments when being loaded into the one or more processing circuitries of the controller 130. The data carrier may be, e.g., a hard disk, a CD ROM disc, a memory stick, an optical storage device, a magnetic storage device or any other appropriate medium such as a disk or tape that may hold machine readable data in a non-transitory manner. The computer program may furthermore be provided as computer program code on a server and downloaded to the controller 130 remotely, e.g. over an Internet or an intranet connection.

The embodiments, or parts thereof, illustrated in Figure 1, Figure 2A, Figure 2B, Figure 3A, Figure 3B, Figure 4A, Figure 4B, Figure 5, Figure 6A and/ or Figure 6B may with advantage be combined with each other for achieving further benefits.

The terminology used in the description of the embodiments as illustrated in the accompanying drawings is not intended to be limiting of the described arrangement 100. Various changes, substitutions and/ or alterations may be made, without departing from invention embodiments as defined by the appended claims.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" and/ or "comprising", specifies the presence of stated features, actions, integers, steps, operations, elements, and/ or components, but do not preclude the presence or addition of one or more other features, actions, integers, steps, operations, elements, components, and/ or groups thereof. A single unit such as e.g. a processor may fulfil the functions of several items recited in the claims. The mere fact that certain measures or features are recited in mutually different dependent claims, illustrated in different figures or discussed in conjunction with different embodiments does not indicate that a combination of these measures or features cannot be used to advantage. A computer program may be stored/ distributed on a suitable medium, such as an optical storage medium or a solid-state medium supplied together with or as part of other hardware, but may also be distributed in other forms such as via Internet or other wired or wireless communication system.

## Claims

1. An arrangement (100) arranged to align a needle (150) and a dry stick (110) in order to apply a milk sample of an animal (101) to the dry stick (110) when the dry stick (110) is positioned in a milk sample apply position in which the needle (150) is aligned with the dry stick (110), wherein the dry stick (110) is configured to indicate at least one biomarker value of the applied milk sample; and wherein the arrangement (100) comprises:
a carrier (115) comprising at least one dry stick (110); wherein the carrier (115) has a longitudinal extension, extending in a Y- direction;
a tube element (155) arranged to receive the milk sample of the animal (101);
a needle (150) connected to the tube element (155), wherein the needle (150) is arranged to receive the milk sample of the animal (101) via the tube element (155) and apply the milk sample to the dry stick (110);
a first light source (160) directed to illuminate the dry stick (110) for creating a first shadow (111) of the dry stick (110) on the carrier (115);
a camera (120) configured to capture an image (300) of at least a part of the dry stick (110) and the first shadow (111) of the part of the dry stick (110) on the carrier (115);
a drive unit (170) comprising an interface (175) configured to cooperate with the carrier (115) in order to move the dry stick (110) on the carrier (115) in the Y- direction to the milk sample apply position;
a controller (130), communicatively connected to the camera (120) and the drive unit (170), **characterised in that** the controller (130) is configured to
trigger the camera (120) to capture the image (300) of the part of the dry stick (110) and the first shadow (111) of the part of the dry stick (110) on the carrier (115);
receive the captured image (300) from the camera (120);
perform an image analysis of the image (300), for detecting the part of the dry stick (110) and the first shadow (111) of the part of the dry stick (110) on the carrier (115);
provide a signal to the drive unit (170) to move the carrier (115) and the dry stick (110) in the Y-direction, into the milk sample apply position based on the image analysis, wherein the needle (150) is enabled to apply the milk sample to the dry stick (110).

2. The arrangement (100) according to claim 1, wherein the controller (130) is configured to perform the image analysis of the image (300) for detecting a first edge (310) of the dry stick (110) by detecting the first shadow (111) of the first edge (310) of the dry stick (110), on the carrier (115).

3. The arrangement (100) according to any one of claim 1 or claim 2, wherein the needle (150) is arranged at a predetermined position (420) in the Y- direction and an X-direction perpendicular to the Y-direction, wherein the predetermined position (420) is known by the controller (130); and wherein the image analysis of the image (300), performed by the controller (130) comprises
detecting a section of a first edge (310) of the dry stick (110) by detecting the first shadow (111) of the part of the dry stick (110) on the carrier (115);
extrapolating an extension (410) of the section of the first edge (310) of the dry stick (110) at a segment (320) of the dry stick (110) where the milk sample is to be applied by the needle (150) when the dry stick (110) is in the milk sample apply position; and
calculating a distance (d) in the Y-direction between the extrapolated extension (410) of the first edge (310) at the segment (320) of the dry stick (110) and the predetermined position (420) of the needle (150); and
generating the milk apply signal to be provided to the drive unit (170) to move the carrier (115) and the dry stick (110) in the Y-direction into the milk sample apply position, based on the calculated distance (d) thereby aligning the predetermined position (420) of the needle (150) with the segment (320) of the dry stick (110) where the milk sample is to be applied by the needle (150).

4. The arrangement (100) according to any one of claims 1-3, wherein the first light source (160) is directed to illuminate the dry stick (110) at an angle of incidence of substantially 45 ±15 degrees.

5. The arrangement (100) according to any one of claims 1-4, wherein the controller (130) have knowledge of the width of the dry stick (110) and is configured to move the carrier (115) and the dry stick (110) in the Y-direction, for aligning the dry stick (110) on which the milk sample is to be applied, with the predetermined position (420) of the needle (150), based on the image processing and with knowledge of the width of the dry stick (110).

6. The arrangement (100) according to any of claims 2-5, comprising a second light source (162) directed to illuminate the dry stick (110) for creating a second shadow (112) of a second edge (330) of the dry stick (110) on the carrier (115); and wherein the controller (130) is configured to
switch off the light of the first light source (160);
ignite the second light source (162);
trigger the camera (120) to capture the image (300) of the second edge (330) of the dry stick (110) and the second shadow (112) of the second edge (330) of the dry stick (110) on the carrier (115);
receive the captured image (300) from the camera (120); and
perform the image analysis of the image (300), for detecting the second edge (330) of the dry stick (110) by detecting the second shadow (112) of the second edge (330) of the dry stick (110), on the carrier (115);
and wherein the controller (130) is configured to move the carrier (115) and the dry stick (110) in the Y-direction, for aligning the dry stick (110) on which the milk sample is to be applied, with the predetermined position (420) of the needle (150), based on the detected first edge (310) of the dry stick (110) and the second edge (330) of the dry stick (110), respectively.

7. The arrangement (100) according to any one of claims 1-6, wherein the dry stick (110) has a thickness that extends in a Z-direction, which Z-direction is perpendicular to the Y-direction; and wherein the first shadow (111) on the carrier (115) is created by light from the first light source (160) and the part of the dry stick (110) extending in the Z-direction; and wherein the image analysis of the image (300) comprises detecting that two or more dry sticks (110) have been stacked onto each other on the carrier (115) in the Z-direction, by:
estimating extension (w) of the first shadow (111) of the part of the dry stick (110) on the carrier (115) in the Y-direction before the milk sample is applied to the dry stick (110);
comparing the estimated shadow extension (w) with an expected shadow extension value; and, when the estimated shadow extension (w) exceeds the expected shadow extension value,
generating a forward signal to be provided to the drive unit (170) to forward the carrier (115) in the Y-direction, to a subsequent dry stick (110) on the carrier (115).

8. The arrangement (100) according to any one of claims 1-7, wherein the dry stick (110) comprises:
a sample pad (210) for receiving the milk sample;
a conjugate pad (230) comprising conjugate (270);
an indication zone (250) with a test line (251) and a control line (252), arranged to indicate the biomarker of the milk sample, based on the conjugate (270);
an absorbent pad (260) for absorbing the milk sample; and
a porous membrane (240) for receiving a capillary flow of the milk sample from the sample pad (210) via the conjugate pad (230) and the indication zone (250) to the absorbent pad (260), thereby forwarding conjugate (270) dispersed into the milk sample to the indication zone (250); and wherein
the image (300) captured by the camera (120) depicts at least a part of the absorbent pad (260), and the first shadow (111) of the part of the absorbent pad (260) on the carrier (115);
the controller (130) is configured to detect unsuccessful flowing of the applied milk sample through the porous membrane (240) to the absorbent pad (260) of the dry stick (110), by:
estimating an extension (w) of the first shadow (111) of the absorbent pad (260) on the carrier (115) in the Y-direction, at a determined time period after having applied the milk sample;
comparing the estimated shadow extension (w) of the first shadow (111) of the absorbent pad (260) on the carrier (115) with an expected reference shadow value; and
discarding the dry stick (110) when the estimated shadow extension (w) of the first shadow (111) of the absorbent pad (260) on the carrier (115) is smaller than the expected reference shadow value.

## Patentansprüche

1. Anordnung (100), die angeordnet ist, um eine Nadel (150) und einen Trockenstreifen (110) auszurichten, um eine Milchprobe eines Tieres (101) auf den Trockenstreifen (110) aufzubringen, wenn der Trockenstreifen (110) in einer Milchprobenaufbringposition positioniert ist, in der die Nadel (150) mit dem Trockenstreifen (110) ausgerichtet ist, wobei der Trockenstreifen (110) konfiguriert ist, um mindestens einen Biomarker-Wert der aufgebrachten Milchprobe anzuzeigen; und wobei die Anordnung (100) umfasst:
einen Träger (115), umfassend mindestens einen Trockenstreifen (110); wobei der Träger (115) eine Längserstreckung aufweist, die sich in einer Y-Richtung erstreckt;
ein Rohrelement (155), das angeordnet ist, um die Milchprobe des Tieres (101) zu empfangen;
eine Nadel (150), die mit dem Rohrelement (155) verbunden ist, wobei die Nadel (150) angeordnet ist, um die Milchprobe des Tieres (101) über das Rohrelement (155) zu empfangen und die Milchprobe auf den Trockenstreifen (110) aufzubringen;
eine erste Lichtquelle (160), die gerichtet ist, um den Trockenstreifen (110) zu beleuchten, zum Erzeugen eines ersten Schattens (111) des Trockenstreifens (110) auf dem Träger (115);
eine Kamera (120), die konfiguriert ist, um ein Bild (300) von mindestens einem Teil des Trockenstreifens (110) und dem ersten Schatten (111) des Teils Trockenstreifens (110) auf dem Träger (115) zu erfassen;
eine Antriebseinheit (170), umfassend eine Schnittstelle (175), die konfiguriert ist, um mit dem Träger (115) zusammenzuwirken, um den Trockenstreifen (110) auf dem Träger (115) in der Y-Richtung zu der Milchprobenaufbringposition zu bewegen;
eine Steuerung (130), die mit der Kamera (120) und der Antriebseinheit (170) kommunikativ verbunden ist, **dadurch gekennzeichnet, dass** die Steuerung (130) konfiguriert ist zum
Auslösen der Kamera (120), um das Bild (300) des Teils des Trockenstreifens (110) und des ersten Schattens (111) des Teils des Trockenstreifens (110) auf dem Träger (115) zu erfassen;
Empfangen des erfassten Bildes (300) von der Kamera (120);
Durchführen einer Bildanalyse des Bildes (300), zum Detektieren des Teils des Trockenstreifens (110) und des ersten Schattens (111) des Teils des Trockenstreifens (110) auf dem Träger (115);
Bereitstellen eines Signals an die Antriebseinheit (170), um den Träger (115) und den Trockenstreifen (110) in der Y-Richtung in die Milchprobenaufbringposition basierend auf der Bildanalyse zu bewegen, wobei die Nadel (150) aktiviert ist, um die Milchprobe auf den Trockenstreifen (110) aufzubringen.

2. Anordnung (100) nach Anspruch 1, wobei die Steuerung (130) konfiguriert ist, um die Bildanalyse des Bildes (300) zum Detektieren einer ersten Kante (310) des Trockenstreifens (110) durch das Detektieren des ersten Schattens (111) der ersten Kante (310) des Trockenstreifens (110) auf dem Träger (115) durchzuführen.

3. Anordnung (100) nach einem der Ansprüche 1 oder 2, wobei die Nadel (150) an einer zuvor bestimmten Position (420) in der Y-Richtung und einer X-Richtung senkrecht zu der Y-Richtung angeordnet ist, wobei die zuvor bestimmte Position (420) der Steuerung (130) bekannt ist; und wobei die Bildanalyse des Bildes (300), durchgeführt durch die Steuerung (130), umfasst
Detektieren eines Abschnitts einer ersten Kante (310) des Trockenstreifens (110) durch das Detektieren des ersten Schattens (111) des Teils des Trockenstreifens (110) auf dem Träger (115);
Extrapolieren einer Erstreckung (410) des Abschnitts der ersten Kante (310) des Trockenstreifens (110) an einem Segment (320) des Trockenstreifens (110), wo die Milchprobe durch die Nadel (150) aufzubringen ist, wenn sich der Trockenstreifen (110) in der Milchprobenaufbringposition befindet; und
Berechnen eines Abstands (d) in der Y-Richtung zwischen der extrapolierten Erstreckung (410) der ersten Kante (310) an dem Segment (320) des Trockenstreifens (110) und der zuvor bestimmten Position (420) der Nadel (150); und
Erzeugen des Milchaufbringsignals, das der Antriebseinheit (170) bereitzustellen ist, um den Träger (115) und den Trockenstreifen (110) in der Y-Richtung in die Milchprobenaufbringposition zu bewegen, basierend auf dem berechneten Abstand (d), wodurch die zuvor bestimmte Position (420) der Nadel (150) mit dem Segment (320) des Trockenstreifens (110) ausgerichtet wird, wo die Milchprobe durch die Nadel (150) aufzubringen ist.

4. Anordnung (100) nach einem der Ansprüche 1 bis 3, wobei die erste Lichtquelle (160) gerichtet ist, um den Trockenstreifen (110) in einem Einfallswinkel von im Wesentlichen 45 ±15 Grad zu beleuchten.

5. Anordnung (100) nach einem der Ansprüche 1 bis 4, wobei die Steuerung (130) Kenntnis von der Breite des Trockenstreifens (110) aufweist und konfiguriert ist, um den Träger (115) und den Trockenstreifen (110) in der Y-Richtung zu bewegen, zum Ausrichten des Trockenstreifens (110), auf den die Milchprobe aufzubringen ist, mit der zuvor bestimmten Position (420) der Nadel (150), basierend auf der Bildverarbeitung und mit Kenntnis der Breite des Trockenstreifens (110).

6. Anordnung (100) nach einem der Ansprüche 2 bis 5, umfassend eine zweite Lichtquelle (162), die gerichtet ist, um den Trockenstreifen (110) zu beleuchten, zum Erzeugen eines zweiten Schattens (112) einer zweiten Kante (330) des Trockenstreifens (110) auf dem Träger (115); und wobei die Steuerung (130) konfiguriert ist zum
Ausschalten des Lichts der ersten Lichtquelle (160);
Entzünden der zweiten Lichtquelle (162);
Auslösen der Kamera (120), um das Bild (300) der zweiten Kante (330) des Trockenstreifens (110) und des zweiten Schattens (112) der zweiten Kante (330) des Trockenstreifens (110) auf dem Träger (115) zu erfassen;
Empfangen des erfassten Bildes (300) von der Kamera (120); und
Durchführen der Bildanalyse des Bildes (300), zum Detektieren der zweiten Kante (330) des Trockenstreifens (110) durch das Detektieren des zweiten Schattens (112) der zweiten Kante (330) des Trockenstreifens (110) auf dem Träger (115);
und wobei die Steuerung (130) konfiguriert ist, um den Träger (115) und den Trockenstreifen (110) in der Y-Richtung zu bewegen, um den Trockenstreifen (110), auf den die Milchprobe aufzubringen ist, mit der zuvor bestimmten Position (420) der Nadel (150) auszurichten, basierend auf der detektierten ersten Kante (310) des Trockenstreifens (110) beziehungsweise der zweiten Kante (330) des Trockenstreifens (110).

7. Anordnung (100) nach einem der Ansprüche 1 bis 6, wobei der Trockenstreifen (110) eine Dicke aufweist, die sich in einer Z-Richtung erstreckt, wobei die Z-Richtung senkrecht zu der Y-Richtung ist; und wobei der erste Schatten (111) auf dem Träger (115) durch Licht von der ersten Lichtquelle (160) und dem Teil des Trockenstreifens (110) erzeugt wird, der sich in der Z-Richtung erstreckenden; und wobei die Bildanalyse des Bildes (300) das Detektieren umfasst, dass zwei oder mehr Trockenstreifen (110) in der Z-Richtung aufeinander auf den Träger (115) gestapelt wurden, durch:
Schätzen der Erstreckung (w) des ersten Schattens (111) des Teils des Trockenstreifens (110) auf dem Träger (115) in der Y-Richtung vor dem Aufbringen der Milchprobe auf den Trockenstreifen (110);
Vergleichen der geschätzten Schattenerstreckung (w) mit einem erwarteten Schattenerstreckungswert; und, wenn die geschätzte Schattenerstreckung (w) den erwarteten Schattenerstreckungswert überschreitet,
Erzeugen eines Weiterleitungssignals, das der Antriebseinheit (170) bereitzustellen ist, um den Träger (115) in der Y-Richtung zu einem nachfolgenden Trockenstreifen (110) auf dem Träger (115) weiterzuleiten.

8. Anordnung (100) nach einem der Ansprüche 1 bis 7, wobei der Trockenstreifen (110) umfasst:
ein Probenkissen (210) zum Empfangen der Milchprobe;
ein Konjugatkissen (230), umfassend das Konjugat (270);
eine Anzeigezone (250) mit einer Testlinie (251) und einer Steuerlinie (252), die angeordnet sind, um den Biomarker der Milchprobe basierend auf dem Konjugat (270) anzuzeigen;
ein Absorptionskissen (260) zum Absorbieren der Milchprobe; und
eine poröse Membran (240) zum Empfangen eines Kapillarflusses der Milchprobe von dem Probenkissen (210) über das Konjugatkissen (230) und die Anzeigezone (250) zu dem Absorptionskissen (260), wodurch Konjugat (270), das in die Milchprobe dispergiert ist, zu der Anzeigezone (250) weitergeleitet wird; und wobei
das Bild (300), das durch die Kamera (120) erfasst wird, mindestens einen Teil des Absorptionskissens (260) und den ersten Schatten (111) des Teils des Absorptionskissens (260) auf dem Träger (115) darstellt;
die Steuerung (130) konfiguriert ist, um ein erfolgloses Fließen der aufgebrachten Milchprobe durch die poröse Membran (240) zu dem Absorptionskissen (260) des Trockenstreifens (110) zu detektieren, durch:
Schätzen einer Erstreckung (w) des ersten Schattens (111) des Absorptionskissens (260) auf dem Träger (115) in der Y-Richtung zu einer bestimmten Zeitspanne nach dem Aufbringen der Milchprobe;
Vergleichen der geschätzten Schattenerstreckung (w) des ersten Schattens (111) des Absorptionskissens (260) auf dem Träger (115) mit einem erwarteten Referenzschattenwert; und
Verwerfen des Trockenstreifens (110), wenn die geschätzte Schattenerstreckung (w) des ersten Schattens (111) des Absorptionskissens (260) auf dem Träger (115) kleiner als der erwartete Referenzschattenwert ist.

## Revendications

1. Agencement (100) agencé pour aligner une aiguille (150) et un bâtonnet sec (110) afin d'appliquer un échantillon de lait d'un animal (101) sur le bâtonnet sec (110) lorsque le bâtonnet sec (110) est positionné dans une position d'application d'échantillon de lait dans laquelle l'aiguille (150) est alignée avec le bâtonnet sec (110), dans lequel le bâtonnet sec (110) est conçu pour indiquer au moins une valeur de biomarqueur de l'échantillon de lait appliqué ; et dans lequel l'agencement (100) comprend :
un support (115) comprenant au moins un bâtonnet sec (110) ; dans lequel le support (115) a une extension longitudinale s'étendant dans une direction Y ;
un élément de tube (155) agencé pour recevoir l'échantillon de lait de l'animal (101) ;
une aiguille (150) reliée à l'élément de tube (155), dans lequel l'aiguille (150) est agencée pour recevoir l'échantillon de lait de l'animal (101) par l'intermédiaire de l'élément de tube (155) et appliquer l'échantillon de lait sur le bâtonnet sec (110) ;
une première source de lumière (160) dirigée pour éclairer le bâtonnet sec (110) pour créer une première ombre (111) du bâtonnet sec (110) sur le support (115) ;
une caméra (120) configurée pour capturer une image (300) d'au moins une partie du bâtonnet sec (110) et de la première ombre (111) de la partie du bâtonnet sec (110) sur le support (115) ;
une unité d'entraînement (170) comprenant une interface (175) conçue pour coopérer avec le support (115) afin de déplacer le bâtonnet sec (110) sur le support (115) dans la direction Y jusqu'à la position d'application d'échantillon de lait ;
un dispositif de commande (130), connecté en communication à la caméra (120) et à l'unité d'entraînement (170), **caractérisé en ce que** le dispositif de commande (130) est configuré pour
déclencher la caméra (120) pour capturer l'image (300) de la partie du bâtonnet sec (110) et la première ombre (111) de la partie du bâtonnet sec (110) sur le support (115) ;
recevoir l'image capturée (300) à partir de la caméra (120) ;
effectuer une analyse d'image de l'image (300), pour détecter la partie du bâtonnet sec (110) et la première ombre (111) de la partie du bâtonnet sec (110) sur le support (115) ;
fournir un signal à l'unité d'entraînement (170) pour déplacer le support (115) et le bâtonnet sec (110) dans la direction Y, jusque dans la position d'application d'échantillon de lait sur la base de l'analyse d'image, dans lequel l'aiguille (150) est activée pour appliquer l'échantillon de lait sur le bâtonnet sec (110).

2. Agencement (100) selon la revendication 1, dans lequel le dispositif de commande (130) est configuré pour effectuer l'analyse d'image de l'image (300) pour la détection d'un premier bord (310) du bâtonnet sec (110) en détectant la première ombre (111) du premier bord (310) du bâtonnet sec (110), sur le support (115).

3. Agencement (100) selon l'une quelconque parmi la revendication 1 ou la revendication 2, dans lequel l'aiguille (150) est agencée au niveau d'une position prédéterminée (420) dans la direction Y et une direction X perpendiculaire à la direction Y, dans lequel la position prédéterminée (420) est connue par le dispositif de commande (130) ; et dans lequel l'analyse d'image de l'image (300), effectuée par le dispositif de commande (130) comprend
la détection d'une section d'un premier bord (310) du bâtonnet sec (110) par la détection de la première ombre (111) de la partie du bâtonnet sec (110) sur le support (115) ;
l'extrapolation d'une extension (410) de la section du premier bord (310) du bâtonnet sec (110) au niveau d'un segment (320) du bâtonnet sec (110) où l'échantillon de lait doit être appliqué par l'aiguille (150) lorsque le bâtonnet sec (110) est dans la position d'application d'échantillon de lait ; et
le calcul d'une distance (d) dans la direction Y entre l'extension extrapolée (410) du premier bord (310) au niveau du segment (320) du bâtonnet sec (110) et la position prédéterminée (420) de l'aiguille (150) ; et
la génération du signal d'application de lait à fournir à l'unité d'entraînement (170) pour déplacer le support (115) et le bâtonnet sec (110) dans la direction Y jusque dans la position d'application d'échantillon de lait, sur la base de la distance calculée (d), alignant ainsi la position prédéterminée (420) de l'aiguille (150) avec le segment (320) du bâtonnet sec (110) où l'échantillon de lait doit être appliqué par l'aiguille (150).

4. Agencement (100) selon l'une quelconque des revendications 1 à 3, dans lequel la première source de lumière (160) est dirigée pour éclairer le bâtonnet sec (110) selon un angle d'incidence de sensiblement 45 ± 15 degrés.

5. Agencement (100) selon l'une quelconque des revendications 1 à 4, dans lequel le dispositif de commande (130) a connaissance de la largeur du bâtonnet sec (110) et est conçu pour déplacer le support (115) et le bâtonnet sec (110) dans la direction Y, pour aligner le bâtonnet sec (110) sur lequel l'échantillon de lait doit être appliqué, avec la position prédéterminée (420) de l'aiguille (150), sur la base du traitement d'image et avec la connaissance de la largeur du bâtonnet sec (110).

6. Agencement (100) selon l'une quelconque des revendications 2 à 5, comprenant une seconde source de lumière (162) dirigée pour éclairer le bâtonnet sec (110) pour créer une seconde ombre (112) d'un second bord (330) du bâtonnet sec (110) sur le support (115) ; et dans lequel le dispositif de commande (130) est configuré pour
éteindre la lumière de la première source de lumière (160) ;
allumer la seconde source de lumière (162) ;
déclencher la caméra (120) pour capturer l'image (300) du second bord (330) du bâtonnet sec (110) et de la seconde ombre (112) du second bord (330) du bâtonnet sec (110) sur le support (115) ;
recevoir l'image capturée (300) à partir de la caméra (120) ; et
effectuer l'analyse d'image de l'image (300), pour détecter le second bord (330) du bâtonnet sec (110) en détectant la seconde ombre (112) du second bord (330) du bâtonnet sec (110), sur le support (115) ;
et dans lequel le dispositif de commande (130) est conçu pour déplacer le support (115) et le bâtonnet sec (110) dans la direction Y, pour aligner le bâtonnet sec (110) sur lequel l'échantillon de lait doit être appliqué, avec la position prédéterminée (420) de l'aiguille (150), sur la base du premier bord (310) détecté du bâtonnet sec (110) et du second bord (330) du bâtonnet sec (110), respectivement.

7. Agencement (100) selon l'une quelconque des revendications 1 à 6, dans lequel le bâtonnet sec (110) a une épaisseur qui s'étend dans une direction Z, laquelle direction Z est perpendiculaire à la direction Y ; et dans lequel la première ombre (111) sur le support (115) est créée par la lumière provenant de la première source de lumière (160) et la partie du bâtonnet sec (110) s'étendant dans la direction Z ; et dans lequel l'analyse d'image de l'image (300) comprend la détection du fait que deux bâtonnets secs (110) ou plus ont été empilés l'un sur l'autre sur le support (115) dans la direction Z, :
en estimant une extension (w) de la première ombre (111) de la partie du bâtonnet sec (110) sur le support (115) dans la direction Y avant que l'échantillon de lait ne soit appliqué sur le bâtonnet sec (110) ;
en comparant l'extension d'ombre estimée (w) à une valeur d'extension d'ombre attendue ; et, lorsque l'extension d'ombre estimée (w) dépasse la valeur d'extension d'ombre attendue,
en générant un signal d'avancement à fournir à l'unité d'entraînement (170) pour faire avancer le support (115) dans la direction Y, vers un bâtonnet sec (110) suivant sur le support (115).

8. Agencement (100) selon l'une quelconque des revendications 1 à 7, dans lequel le bâtonnet sec (110) comprend :
un tampon d'échantillon (210) pour recevoir l'échantillon de lait ;
un tampon de conjugué (230) comprenant un conjugué (270) ;
une zone d'indication (250) avec une ligne de test (251) et une ligne de commande (252), agencée pour indiquer le biomarqueur de l'échantillon de lait, sur la base du conjugué (270) ;
un tampon absorbant (260) pour absorber l'échantillon de lait ; et
une membrane poreuse (240) pour recevoir un écoulement capillaire de l'échantillon de lait à partir du tampon d'échantillon (210) par l'intermédiaire du tampon de conjugué (230) et la zone d'indication (250) vers le tampon absorbant (260), faisant ainsi avancer le conjugué (270) dispersé dans l'échantillon de lait vers la zone d'indication (250) ; et dans lequel
l'image (300) capturée par la caméra (120) représente au moins une partie du tampon absorbant (260), et la première ombre (111) de la partie du tampon absorbant (260) sur le support (115) ;
le dispositif de commande (130) est configuré pour détecter un écoulement infructueux de l'échantillon de lait appliqué à travers la membrane poreuse (240) vers le tampon absorbant (260) du bâtonnet sec (110), :
en estimant une extension (w) de la première ombre (111) du tampon absorbant (260) sur le support (115) dans la direction Y, à une période de temps déterminé après avoir appliqué l'échantillon de lait ;
en comparant l'extension d'ombre estimée (W) de la première ombre (111) du tampon absorbant (260) sur le support (115) avec une valeur d'ombre de référence attendue ; et
en rejetant le bâtonnet sec (110) lorsque l'extension d'ombre estimée (w) de la première ombre (111) du tampon absorbant (260) sur le support (115) est inférieure à la valeur d'ombre de référence attendue.
